# EUROPÄISCHE PATENTSCHRIFT

(11) **EP 1 123 266 B1**
(45) Veröffentlichungstag und Bekanntmachung des Hinweises auf die Patenterteilung: **28.05.2003**
(21) Anmeldenummer: 99950745.2
(22) Anmeldetag: 15.10.1999
(51) Int. Cl.: C07C 51/43, C07C 65/24

(54) **VERFAHREN ZUR TROCKNUNG VON PHENOXYMETHYLBENZOESÄUREN**
METHOD FOR DRYING PHENOXYMETHYLBENZOIC ACIDS
PROCEDE DE SECHAGE D'ACIDES PHENOXYMETHYLBENZOIQUES

(30) Priorität: 20.10.1998 DE 19848200
(43) Veröffentlichungstag der Anmeldung: 16.08.2001
(73) Patentinhaber: BASF AKTIENGESELLSCHAFT, 67056 Ludwigshafen (DE)
(72) Erfinder: ISAK, Heinz, D-67459 Böhl-Iggelheim (DE); LAMBERT, Martin, D-68163 Mannheim (DE)
(86) Internationale Anmeldenummer: EP9907826
(87) Internationale Veröffentlichungsnummer: WO00023413

(56) Entgegenhaltungen:
- EP-A- 0 712 833
- WO-A-98/14420

## Beschreibung

Die vorliegende Erfindung betrifft ein Verfahren zur Trocknung von Phenoxymethylbenzoesäuren der allgemeinen Formel I 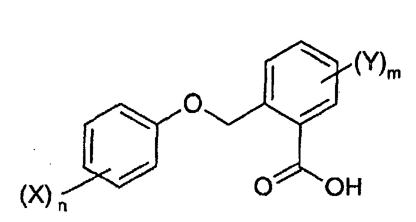 wobei X, Y, m und n die folgenden Bedeutungen haben:
- X,Y: Halogen oder Methyl, Ethyl, i- und n-Propyl, sowie Butyl,
- m: einen Wert von 0 bis 4 und
- n: einen Wert von 0 bis 5

Phenoxymethylbenzoesäuren der Formel I sind wertvolle Zwischenprodukte für die Herstellung von fungiziden Wirkstoffen.

In der DE 27 49 957 wird ein Verfahren zur Herstellung u.a. von Verbindungen der Formel I aus Phthalid- und Phenol-Derivaten beschrieben. Die erzielten Ausbeuten können jedoch nicht zufriedenstellen und die erhaltenen Produkte neigen nach dem Trocknen zum Verkleben und Verbacken.

Nach dem Verfahren der EP-A 493 711 erhält man deutlich höhere Ausbeuten, doch zeigen die danach erhältlichen Produkte ein schlechtes Fließverhalten, und durch Brückenbildung tritt eine Verfestigung ein. Eine technische Realisierung des Verfahrens wird dadurch erschwert, da die Feststoffverarbeitung bzw. der Transport des Feucht- und Trockengutes sowie deren Lagerung problematisch sind. Hinzu kommt, daß bei der Trocknung staubexplosionsfähige Gemische entstehen können. Diese Probleme machen es erforderlich, bei der Durchführung des Verfahrens einen erheblichen technischen Aufwand zu betreiben, der das Verfahren insgesamt in seiner Wirtschaftlichkeit deutlich beeinträchtigt.

Der vorliegenden Erfindung lag daher die Aufgabe zugrunde, ein Verfahren zur Trocknung von Phenoxymethylbenzoesäuren der Formel I zur Verfügung zu stellen, welches die geschilderten Nachteile nicht aufweist und auf einfache Weise ein gut weiter zu verarbeitendes Produkt liefert.

Erfindungsgemäß wird diese Aufgabe gelöst durch ein Verfahren gemäß Anspruch 1, wobei die Trocknung von wasser- und/oder lösungsmittelfeuchten Phenoxymethylbenzoesäuren in flüssiger Phase durchgeführt wird.

Die Phenoxymethylbenzoesäuren haben die allgemeine Formel 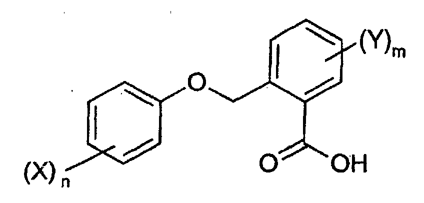 wobei X, Y, m und n die folgenden Bedeutungen haben:
- X,Y: Halogen oder Methyl, Ethyl, i- und n-Propyl, sowie Butyl,
- m: einen Wert von 0 bis 4 und
- n: einen Wert von 0 bis 5.

Halogen kann dabei für Cl, Br, I oder F, bevorzugt Cl oder F stehen.

n und m haben vorzugsweise einen Wert im Bereich von 0 bis 3, vorzugsweise 0 oder 1.

Bevorzugte Reste X und Y sind Methyl und Ethyl.

Überraschenderweise wird nach dem erfindungsgemäßen Verfahren ein Produkt mit einer niedrigen Viskosität erhalten, welches gut weiter verarbeitet werden kann.

Nach einer vorteilhaften Ausführungsform des Verfahrens wird die Trocknung bei Temperaturen in einem Bereich von 1 bis 25, besonders bevorzugt 3-20 °C oberhalb des Schmelzpunktes der Phenoxymethylbenzoesäuren unter den angewandten Reaktionsbedingungen (Temperatur, Druck) durchgeführt. Bei Normaldruck führt dies zu Trocknungstemperaturen im Bereich von etwa 130 bis 240 °C.

Auch die Tatsache, daß die Phenoxymethylbenzoesäuren trotz der anzuwendenden hohen Temperaturen oberhalb des Schmelzpunktes keine Decarboxylierungs- oder Spaltreaktionen eingehen, war nicht von vornherein zu erwarten.

Ein weiterer Vorteil des erfindungsgemäßen Verfahrens liegt in der einfachen technischen Realisierbarkeit, da allgemein verwendete Standardreaktoren verwendet werden können; es ist lediglich eine exakte Temperaturregelung erforderlich.

Bevorzugt wird das erfindungsgemäße Verfahren in Reaktoren mit einer Durchmischungsmöglichkeit, z.B. Rührreaktoren durchgeführt. Durch das Umwälzen bzw.Rühren der flüssigen Phase kann die Oberfläche vergrößert werden, was den Wirkungsgrad des erfindungsgemäßen Verfahrens erhöht.

Falls erforderlich kann die Trocknung nach dem erfindungsgemäßen Verfahren auch unter inerten Bedingungen unter Schutzgas erfolgen, wenn die Stabilität der zu trocknenden Phenoxymethylbenzoesäure dies erforderlich macht.

Durch Anlegen von Vakuum unterschiedlichen Grades kann die Schmelztemperatur der eingesetzten Phenoxymethylbenzoesäuren und damit auch die Temperatur, bei der das erfindungsgemäße Verfahren durchgeführt wird, verringert und damit die Trocknung unterstützt werden. Dies kann in einigen Fällen von Vorteil sein.

Falls die eingesetzten Phenoxymethylbenzoesäuren Wasser und Lösungsmittel enthalten und der Siedepunkt des Lösungsmittels sich von der des Wassers erheblich unterscheidet, kann die Trocknung auch zweistufig durchgeführt werden, das heißt, es wird zunächst die niedriger siedende Komponente und anschließend die höher siedende Komponente entfernt. Die Zweistufigkeit kann dabei sowohl über einen Temperaturgradienten als auch über einen Druckgradienten bei konstanter Temperatur erreicht werden. Selbstverständlich ist es auch möglich, sowohl den Druck als auch die Temperatur im Verlauf des Verfahrens zu ändern. Wesentlich ist lediglich, daß der flüssige Zustand der zu trocknenden Phenoxymethylbenzoesäuren nicht verlassen wird.

Eine mehrstufige Trocknung kann sowohl in einem als auch in mehreren Reaktoren durchgeführt werden; bei kontinuerlicher Durchführung einer mehrstufigen Verfahrensvariante ist in der Regel die Durchführung in mehreren Reaktoren vorteilhaft, die jeweils unter konstanten Bedingungen betrieben werden. Bei diskontinuierlicher Verfahrensweise kann dagegen in einem Reaktor einfach ein Temperatur. bzw. Druckgradientenprogramm im Über- und Unterdruckbereich eingestellt werden.

Bevorzugt wird das Verfahren kontinuierlich durchgeführt.

Der Zeitbedarf für die Durchführung des erfindungsgemäßen Verfahrens liegt im allgemeinen im Bereich von 1 min. bis 24 h, vorzugsweise von 1 min. bis 10h. Er hängt unter anderem von der Trocknungstemperatur, der für die Trocknung zur Verfügung stehenden aktiven Oberfläche und dem anfänglichen Wasser- und/oder Lösungsmittelgehalt ab.

Der Energieaufwand für das Aufschmelzen der Phenoxymethylbenzoesäuren erwies sich als geringer als erwartet, da die Schmelzwärme dieser Verbindungen sehr niedrig liegt. Sie liegt im allgemeinen im Bereich von 50 bis 400 kJ/kg.

Die Herstellung der wasser- oder lösungsmittelfeuchten Phenoxymethylbenzoesäuren kann nach an sich bekannten und in der Literatur beschriebenen Verfahren erfolgen. Nähere Angaben erübrigen sich daher hier.

Der Wasser- und/oder Lösungsmittelgehalt der Phenoxymethylbenzoesäuren vor der Durchführung des erfindungsgemäßen Verfahrens liegt in der Regel im Bereich von 0,1 bis 50, vorzugsweise von 5 bis 30 Gew.%. Nach Durchführung des erfindungsgemäßen Verfahrens liegen die Wasser- und/oder Lösungsmittelgehalte noch im Bereich von 0,01 bis 3 , vorzugsweise von 0,01 bis 2 Gew.%.

Enthalten die zu trocknenden Phenoxymethylbenzoesäuren noch Verunreinigungen, deren Siedepunkte im Bereich des Wassers bzw. des enthaltenen Lösungsmittels liegen, wird deren Anteil durch das erfindungsgemäße Verfahren ebenfalls deutlich verringert und es wird ein reineres Produkt erhalten.

Die nach dem erfindungsgemäßen Verfahren erhältlichen Phenoxymethylbenzoesäuren lassen sich sehr gut zu fungiziden Wirkstoffen weiterverarbeiten. Sie weisen im erstarrten Zustand eine deutlich reduzierte (praktisch keine) Neigung zum Verkleben und Verbacken und eine deutlich reduzierte (praktisch keine) Neigung zur Verfestigung durch Brückenbildung auf.

### Beispiele

### Allgemeine Durchführung

In den nachfolgenden Beispielen wurde die wasserfeuchte 2'-Methyl-(2-phenoxymethyl)benzoesäure portionsweise in einen auf etwa 160 °C temperierten Rührreaktor eingetragen, in dem sich als holdup etwa 10 % der eingebrachten Menge aus einem vorhergehenden Ansatz befanden. Abhängig von der einzubringenden Menge (Chargengröße) erfolgte die Einbringung unter Rühren in einem Zeitraum von 1 bis 13 h. In dieser Zeit wurde die Säure auch aufgeschmolzen. Das anfallende Destillat wurde kondensiert. Nach beendeter Zugabe wurde noch eine Stunde nachgerührt und anschließend die Reinheit und der Restwassergehalt aus der homogenen Probe bestimmt.

### Beispiel 1

660 g einer wasserfeuchten 2'-Methyl-(2-phenoxymethyl)benzoesäure mit einem Wassergehalt von 9,6 Gew.% (63,6 g), einem Methanolgehalt von 0,27 Gew.% (1,8 g) und einem Nebenproduktgehalt von 2,5 Gew.% (16,5 g) wurden in den Schmelzreaktor eingetragen.

Nach einer mittleren Verweilzeit von 8 h bei einer Temperatur von 158 bis 163 °C wurde die Schmelze ausgetragen und abgekühlt. Es wurden 595,1 g eines Produkts mit folgender Zusammensetzung erhalten:

| | |
|---|---|
| 97,1 Gew.% (578,1 g) | 2'-Methyl-(2-phenoxymethyl)benzoesäure |
| 0,087 Gew.% (0,52 g) | Wasser |
| 0,015 Gew.% (0,09 g) | Methanol |
| 2,77 Gew.% (16,5 g) | Nebenprodukte |

### Beispiel 2

415,9 g einer wasserfeuchten 2'-Methyl-(2-phenoxymethyl)benzoesäure mit einem Wassergehalt von 16,6 Gew.% (69,1 g), einem Methanolgehalt von 0,12 Gew.% (0,5 g) und einem Nebenproduktgehalt von 1,5 Gew.% (6,3 g) wurden in den Schmelzreaktor eingetragen.

Nach einer mittleren Verweilzeit von 8 h bei einer Temperatur von 158 bis 165 °C wurde die Schmelze ausgetragen und abgekühlt. Es wurden 345,7 g eines Produkts mit folgender Zusammensetzung erhalten:

| | |
|---|---|
| 98,1 Gew.% (339,0 g) | 2'-Methyl-(2-phenoxymethyl)benzoesäure |
| 0,03 Gew.% (0,11 g) | Wasser |
| 0,02 Gew.% (0,08 g) | Methanol |
| 1,9 Gew.% (6,5 g) | Nebenprodukte |

### Beispiel 3

0,7 kg einer ca. 10 Gew.% Methanol enthaltenden 2'-Methyl-(2-phenoxymethyl)benzoesäure wurde 3 bis 4 mal mit je 200 ml Wasser gewaschen und so ein Restmethanolgehalt von weniger als 1 Gew.% erhalten, die anschließend getrocknet wurde.

633,2 g dieser wasserfeuchten 2'-Methyl-(2-phenoxymethyl)benzoesäure mit einem Wassergehalt von 3,8 Gew.% (24,2 g), einem Methanolgehalt von 0,66 Gew.% (4,1 g) und einem Nebenproduktgehalt von 2,0 Gew.% (12,9 g) wurden in den Schmelzreaktor eingetragen.

Nach einer mittleren Verweilzeit von 7 h bei einer Temperatur von 158 bis 163 °C wurde die Schmelze ausgetragen und abgekühlt. Es wurden 606,3 g eines Produkts mit folgender Zusammensetzung erhalten:

| | |
|---|---|
| 97,5 Gew.% (591,0 g) | 2'-Methyl-(2-phenoxymethyl)benzoesäure, |
| 0,052 Gew.% (0,32 g) | Wasser |
| 0,018 Gew.% (0,1 g) | Methanol |
| 2,48 Gew.% (14,9 g) | Nebenprodukte |

## Patentansprüche

1. Verfahren zur Trocknung von Phenoxymethylbenzoesäuren der allgemeinen Formel I 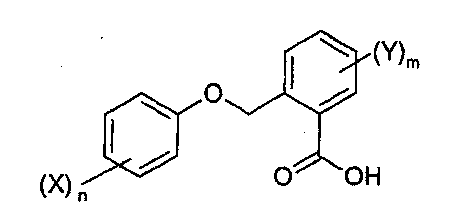 wobei X, Y, m und n die folgenden Bedeutungen haben:
X,Y Halogen oder Methyl, Ethyl, i- und n-Propyl, sowie Butyl,
m einen Wert von 0 bis 4 und
n einen Wert von 0 bis 5
**dadurch gekennzeichnet, daß** man die wasser- und/oder lösungsmittelfeuchten Phenoxymethylbenzoesäuren bei einer Temperatur im Bereich von 1° bis 25 °C oberhalb ihres Schmelzpunktes unter den angewandten Reaktionsbedingungen trocknet.

2. Verfahren nach Anspruch 1, **dadurch gekennzeichnet, daß** man die Trocknung bei Temperaturen im Bereich von 130° bis 240°C unter Normaldruck durchführt.

3. Verfahren nach einem der Ansprüche 1 bis 2, **dadurch gekennzeichnet, daß** man vor der Trocknung Lösungsmittelreste teilweise durch Waschen mit Wasser entfernt.

4. Verfahren nach einem der Ansprüche 1 bis 3, **dadurch gekennzeichnet, daß** man eine Phenoxymethylbenzoesäure mit einem Wasser- und/oder Lösungsmittelgehalt von 0,1 bis 50 Gew.% einsetzt.

## Claims

1. A process for drying phenoxymethylbenzoic acids of the general formula I 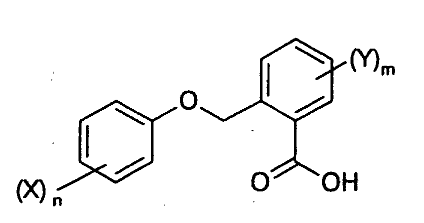 where X, Y, m and n have the following meanings:
X,Y halogen or methyl, ethyl, i- and n-propyl, and butyl,
m a value from 0 to 4 and
n a value from 0 to 5
which comprises drying the water- and/or solvent-wet phenoxymethylbenzoic acids at a temperature in the range from 1° to 25°C above their melting point under the reaction conditions used.

2. A process as claimed in claim 1, wherein the drying is carried out at temperatures in the range from 130° to 240°C under atmospheric pressure.

3. A process as claimed in either of claims 1 and 2, wherein solvent residues are partly removed by washing with water before drying.

4. A process as claimed in any of claims 1 to 3, wherein a phenoxymethylbenzoic acid with a water and/or solvent content of from 0.1 to 50% by weight is employed.

## Revendications

1. Procédé pour le séchage d'acides phénoxyméthylbenzoïques de formule générale I 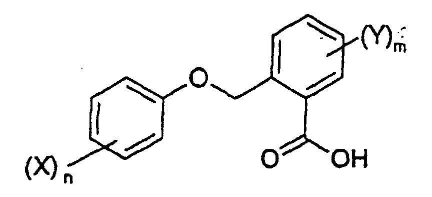 où X, Y, m et m ont les significations suivantes:
X,Y halogéno ou méthyle, éthyle, iso- et n-propyle, ainsi que butyle,
m une valeur de 0 à 4 et
n une valeur de 0 à 5
**caractérisé par le fait qu'**on sèche les acides phénoxyméthylbenzoïques imprégnés d'eau et/ou de solvant à une température dans l'intervalle de 1° à 25°C, au-dessus de leur point de fusion dans les conditions de réaction mises en oeuvre.

2. Procédé selon la revendication 1, **caractérisé par le fait qu'**on effectue le séchage à des températures dans l'intervalle de 130° à 240°C sous pression normale.

3. Procédé selon l'une des revendications 1 à 2, **caractérisé par le fait qu'**avant le séchage on élimine partiellement les restes de solvant par lavage avec de l'eau.

4. Procédé selon l'une des revendications 1 à 3, **caractérisé par le fait qu'**on utilise un acide phénoxyméthylbenzoïque ayant une teneur en eau et/ou en solvant de 0,1 à 50 % en poids.
